# EUROPEAN PATENT SPECIFICATION

(11) **EP 0 345 155 B1**
(45) Date of publication and mention of the grant of the patent: **10.08.1994**
(21) Application number: 89401495.0
(22) Date of filing: 31.05.1989
(51) Int. Cl.: C12N 15/62, C12P 21/00

(54) **Process for producing natural human apolipoprotein e-like proteins**
Verfahren zur Herstellung von Proteinen, die ähnlich dem natürlich-menschlichen Apolipoprotein-E sind
Procédé de préparation des protéines similaires à l'apolipoprotéine-E humaine naturelle

(30) Priority: 31.05.1988 JP 133549/88; 26.04.1989 JP 107027/89
(43) Date of publication of application: 06.12.1989
(73) Proprietor: MITSUBISHI KASEI CORPORATION, Tokyo 100 (JP)
(72) Inventor: Shibui, Tatsurou, Machida-shi Tokyo (JP); Kamizono, Michiru, Machida-shi Tokyo (JP); Teranishi, Yutaka, Sagamihara-shi Kanagawa-ken (JP)
(74) Representative: Gutmann, Ernest

(56) References cited:
- EP-A- 0 111 814
- EP-A- 0 138 644
- EP-A- 0 152 830
- EP-A- 0 154 576
- EP-A- 0 205 715
- EP-A- 0 293 357
- PATENT ABSTRACTS OF JAPAN, vol. 10, no. 276 (C-373) 10 September 1986
- PATENT ABSTRACTS OF JAPAN, vol. 9, no. 267 (C-310) 24 October 1985

## Description

### Field of the Invention

The present invention relates to a microbial process for producing a natural-type human apolipoprotein E-like protein by recombinant DNA techniques.

### Description of the Prior Art

Major lipids present in blood plasma include cholesterol, phospholipids, triglycerides, and free fatty acids. The former three lipids are conjugated with proteins, so that the water-insoluble lipids are solubilized in plasma. Such lipid-protein complexes, which are called "lipoproteins", have various molecular weights depending on their lipid/protein ratios. Lipoproteins have spherical particulate structures which may be considered to be composed of a core of non-polar triglycerides or cholesterol esters and a surface layer formed from polar phospholipids or free cholesterol in association with proteins.

Proteins constituting lipoproteins are called "apolipoproteins". At present, there are known ten or more apolipoproteins. Apolipoproteins are not only essential constituents of lipoproteins but also play an important role in the metabolism of lipoproteins.

Apolipoprotein E is one of the known apolipoproteins. It is known that apolipoprotein E functions as a recognition marker when cells in a living body incorporate lipoproteins through their receptors. Further, it has also been found that apolipoprotein E has three main subclasses E2, E3 and E4. Apolipoprotein E3 was derived from normal human beings, while the other subclasses, E2 and E4, were found in patients with hyperlipidemia type III and are considered to be of abnormal type: The Journal of Biological Chemistry, Vol. 255, No. 5, 1759-1762 (1980).

Those who are deficient in the apolipoprotein E3, or those who have the apolipoprotein E2 or E4, often suffer from hyperlipidemia resulting in arteriosclerosis. The normal functions of the apolipoprotein E in such patients can be recovered by administration of the normal apolipoprotein E3.

The present inventors found the full length cDNA fragment coding for the apolipoprotein E (Japanese Patent Application Laying-open KOKAI No. 118189/1985), and proposed a process for producing the apolipoprotein E in Escherichia coli: refer to Japanese Patent Application Laying-open KOKAI No. 96997/1986. The apolipoprotein E produced by said process, however, had an additional amino acid, for example, methionine corresponding to the initiation codon.

There has been no report that a protein having a higher molecular weight similar to that of apolipoprotein E could be produced and secreted in a large amount in E. coli as a host. A sole report is large scale secretory production of human growth hormone having a molecular weight, 20 kd, smaller than that of apolipoprotein E: Hsuing et al., Bio/Technology, Vol. 4, 991 (1986).

### SUMMARY OF THE INVENTION

The present inventors have further studied and finally found that human apolipoprotein E having the naturally occurring N-terminal amino acid sequence can microbially be produced and secreted in large amounts by constructing and employing a vector into which a DNA fragment coding for a secretory signal peptide is introduced. Thus, the present invention has now been attained.

Accordingly, it is a primary object of the present invention to provide a microbial process for producing natural human apolipoprotein E or natural human apolipoprotein E-like proteins having physiological activities similar to those of the natural human apolipoprotein E. The term "human apolipoprotein E-like protein" used herein may sometimes encompass both the natural human apolipoprotein E and the human apolipoprotein E-like proteins having physiological activities similar to those of the natural human apolipoprotein E.

According to the present process, the product proteins are not exposed to the action of microbial proteases in a host microorganism and, therefore, human apolipoprotein E-like proteins can be obtained efficiently in larger amounts as much as about 1,000 times or more as compared with conventional processes.

There is provided by the present invention a process for producing natural human apolipoprotein E or a natural human apolipoprotein E-like protein having physiological activities similar to those of the natural human apolipoprotein E, which comprises transforming a host microorganism with an expression vector into which a first DNA fragment coding for a secretory signal peptide and a second DNA fragment coding for the human apolipoprotein E or human apolipoprotein E-like protein are introduced downstream from a promoter; culturing the transformant; and collecting the natural human apolipoprotein E or natural human apolipoprotein E-like protein produced.

### BRIEF DESCRIPTION OF THE DRAWINGS

Several drawings are attached hereto for better understanding the description of the present invention given hereinbelow.

Fig. 1 is a schematic drawing of the vector pOFAapoE constructed in Example 1 according to the present invention.

Fig. 2 is a schematic drawing of the vector pNapoE constructed in Example 2 according to the present invention.

Figs. 3A-3E schematically illustrate the construction of the vector pOFAapoE.

Figs. 4A and 4B show the variant primers used in the construction of the vector pOFAapoE.

Figs. 5A and 5B schematically illustrate the construction of the vector pMTI2 used in Example 1.

Fig. 6 is a schematic drawing of the vector phMAVD.lacI used in Example 1.

Figs. 7A-7D schematically illustrate the construction of the vector pNapoE.

Fig. 8 shows the variant primer used in the construction of the vector pNapoE.

Fig. 9 schematically illustrates the construction of the vector pOFAapoE₂.

Fig. 10 schematically illustrates the construction of the vector pNapoE₂.

### DESCRIPTION OF THE INVENTION

The present invention will further be described in detail.

DNA fragments (the second DNA fragments) used in the present invention which contain a DNA sequence coding for a human apolipoprotein E-like protein, including human apolipoprotein E2, E3 and E4, may be prepared in the following manner.

Human-derived specimens, for example, liver sections, small intestine epidermal cells, blood macrophages or kidney sections, are homogenized in the presence of guanidine isothiocyanate and subjected to CsCl equilibrium density gradient ultracentrifugation to separate total RNA: Chirgwin et al., Biochemistry, 18, 5294-5299 (1979). The total RNA is then purified by conventional oligo(dT) cellulose column chromatography to isolate poly(A)-containing RNAs which are used as mRNA materials in later steps.

The mRNA materials are treated by the method of Okayama and Berg to prepare a cDNA library: Molecular and Cellular Biology, 2, 161-170 (1982). A vector primer and an oligo(dG)-tailed linker are obtained from a hybrid plasmid of pBR322 and SV40. The vector primer and the mRNA materials are used to synthesize cDNAs in the presence of a reverse transcriptase. The cDNAs are digested with HindIII and then cyclized together with the linker. Subsequently, the mRNA portions are substituted by DNAs to yield cDNA fragment-containing plasmids.

These plasmids are then used to transform a microorganism such as Escherichia coli in conventional manners. The resulting ampicillin-resistant transformants are screened using, as a probe, a synthetic oligonucleotide which contains a portion or the whole of the base sequence corresponding to the amino acid sequence 218-222 of apolipoprotein E, Met-Glu-Glu-Met-Gly, or another synthetic oligonucleotide containing at least said base sequence. Thus, clones containing a base sequence complementary to said base sequence are selected. Plasmids from the selected clones are treated with suitable restriction enzymes to select those clones containing a desired plasmid into which the longest cDNA has been inserted.

The base sequence of the cDNA fragment obtained from the selected clones is determined by the method of Maxam and Gilbert: Methods in Enzymology, 65, 499-560 (1980).

DNA fragments (the first DNA fragments) coding for a secretory signal peptide used herein may include, for example, E. coli-derived ones, such as OmpF, OmpC, OmpA, alkaline phosphatase, lipoprotein, and AmpC-beta-lactamase; Bacillus-derived ones, such as alpha-amylase, subtilisin, and neutral protease; yeast-derived ones, such as amylase; and animal cell-derived ones, such as apoE, apoA-I, interferon and HSA.

E. coli-derived DNA fragments coding for a signal peptide are preferred. More preferably, E. coli fragments in which a DNA sequence in the vicinity of the initiation codon for the encoded signal peptide, typically 6 bases in both sides of the codon, is appropriately modified so that the content of adenine (A) and thymine (T) may be increased without changing the amino acids of the signal peptide encoded downstream from the initiation codon. Such modified DNA fragments provide higher expression efficiencies.

Illustratively, such modified DNA sequences may be 5′-AAGCTTATGATGAAG-3′, 5′-AAATTTATGAAG-3′, 5′-AAGCTTATGATGAAA-3′ or 5′-AAATTTATGAAA-3′ for the OmpF signal peptide; and 5′-AAGCTTATGGTT-3′, 5′-AAATTTATGGTT-3′, 5′-AAGCTTATGGTA-3′ or 5′-AAATTTATGGTA-3′ for the OmpC signal peptide.

Expression vectors into which the DNA fragments coding for a human apolipoprotein E-like protein and a secretory signal peptide are incorporated have a promoter at such a position as enabling the control of transcription of these genes.

Any known promoters capable of functioning in host microorganisms may be used herein. Preferred promoters include tac promoter, lambda P_{L}P_{R} promoter, and hybrid promoters which comprise the RNA polymerase recognition region of a phage promoter and the RNA polymerase binding region of an E. coli promoter as disclosed in Japanese Patent Application Laying-open KOKAI No. 123383/1988.

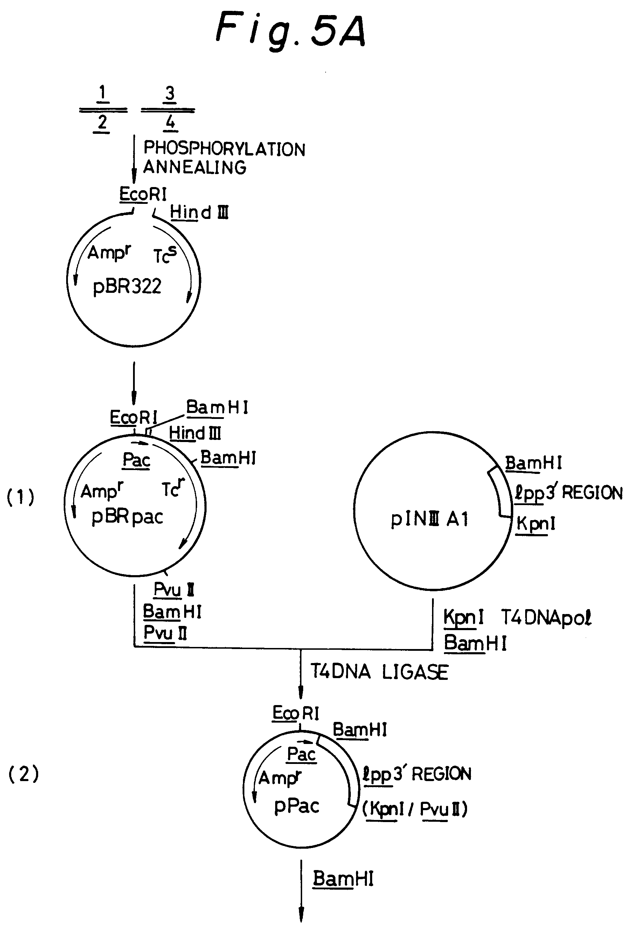

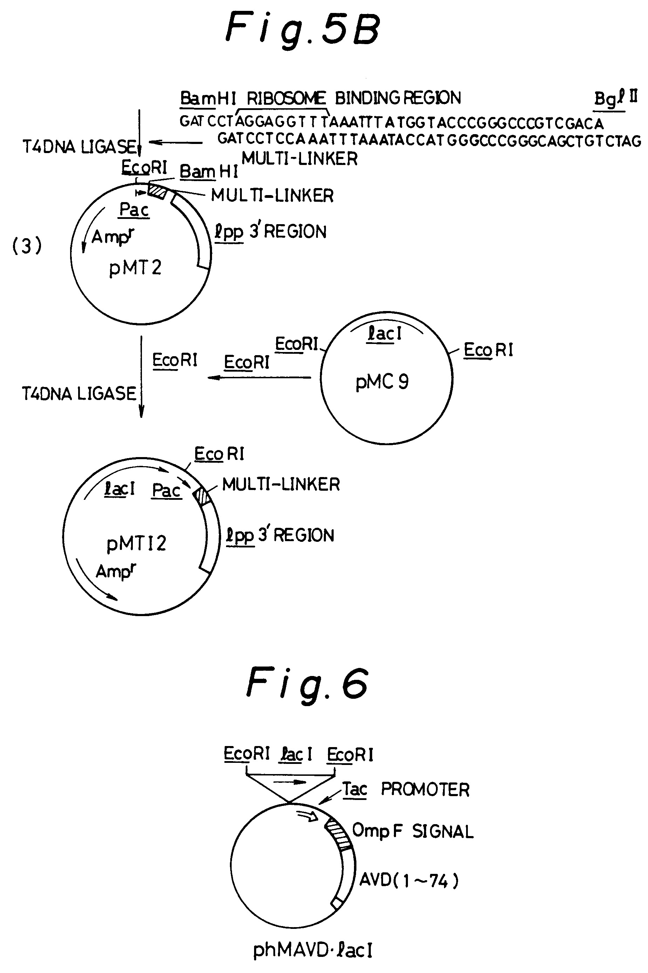

Especially preferred is tac promoter, or the hybrid promoter (pac promoter) which comprises the RNA polymerase recognition region having the base sequence represented by the following formula (I): 
TTGCT ... ... ... (I)
AACGA
and the RNA polymerase binding region having the base sequence represented by the following formula (II) or (III): 
TATAATG
ATATTAC ... ... ... (II)
TATAATGTGTGG
ATATTACACACC ... ... ... (III)
Illustrative examples of such expression vectors may include pMTI2 (Fig. 5) as disclosed in Japanese Patent Application Laying-open KOKAI No. 123383/1988.

According to the present invention, it is preferred to use two or more, particularly two or three promoters as above mentioned which are linked together in tandem to increase the expression efficiency.

The DNA fragments coding for the secretory signal peptide and human apolipoprotein E-like protein may be incorporated into the expression vector by any combination of known methods, as illustrated in the examples described hereinafter.

According to the present invention, it is preferred that two or more, particularly two or three genes coding for the secretory signal peptide and human apolipoprotein E-like protein, which are linked in tandem, are incorporated into the expression vector to increase the expression efficiency.

The expression vectors of the present invention have a ribosome binding region, preferably derived from lac, lpp, trp, or lambda-CII gene.

In addition, the expression vectors of the present invention may preferably have such a DNA sequence that may be substantially complementary to the 3′ terminal base sequence of ribosomal RNA, for example, 5′-AGGAGGTTT-3′, 5′-AGGGTTT-3′, 5′-AGGAAACTGA-3′, 5′-TGGATTATTC-3′- or 5′-AGGAGGTATA-3′.

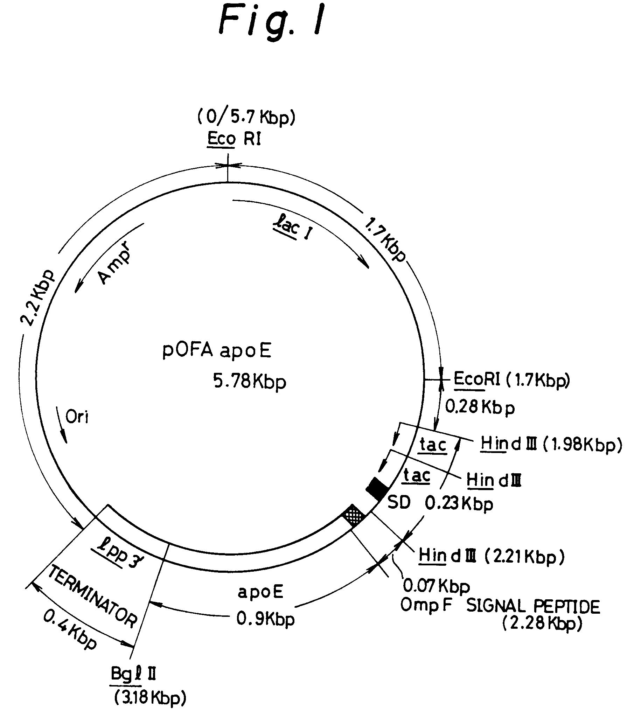

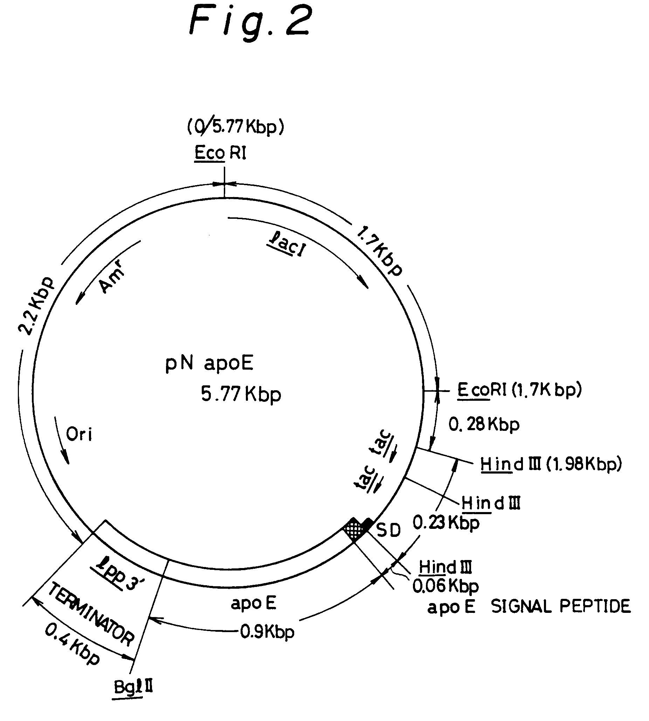

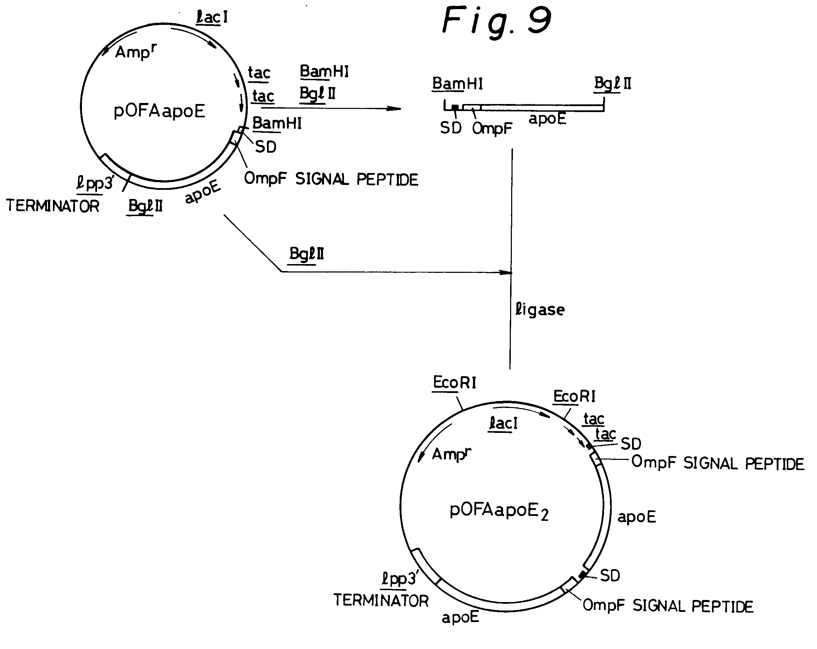

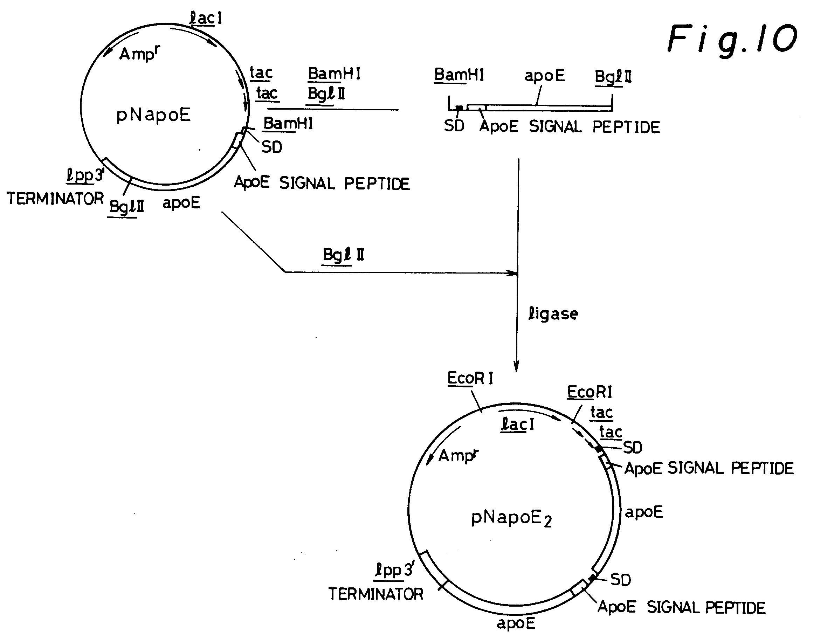

Illustrative examples of such expression vectors according to the present invention may include the vectors pOFAapoE and pNapoE as shown in Figs. 1 and 2, respectively; and the vectors pOFAapoE₂ and pNapoE₂ as shown in Figs. 9 and 10, respectively.

In the present invention, such an expression vector is employed to transform a host microorganism.

Host microorganisms used herein may include E. coli JM109, HB101, YK537 and YA21, and Bacillus MI112 and NP58.

Transformation may be effected according to any conventional method, for example, the method described in Molecular Cloning, Cold Spring Harbor, pp. 250 (1982).

The resulting transformants may then be cultured in M9 minimal, L broth, or other similar media in conventional manners.

According to the present invention, a human apolipoprotein E-like protein may be expressed along with a secretory signal peptide in the transformant. The secretory signal peptide portion may be cleaved upon transportation of the fused peptide through the cell membrane. Thus, the human apolipoprotein E-like protein having the natural N-terminal may be accumulated in the periplasm or out of the cell.

In addition, desired proteins may be produced in larger amounts according to the present invention, since they are secreted from host cells and therefore are not subject to degradation by cellular enzymes.

Illustratively, human apolipoprotein E or a human apolipoprotein E-like protein can be produced in such a large amount as about 50 mg/l according to the present invention. Further, the proteins will be able to be produced in still larger amounts as much as about 450 mg/l when the transformants incorporating the expression vector of the present invention are subjected to high density cultivation in a jar fermentor or similar equipment.

### DESCRIPTION OF THE PREFERRED EMBODIMENTS

The present invention will be more fully illustrated by the following examples, which are not construed as limiting the invention. Many modifications and variations can be made by those skilled in the art without departing from the scope of the present invention as defined in the attached claims.

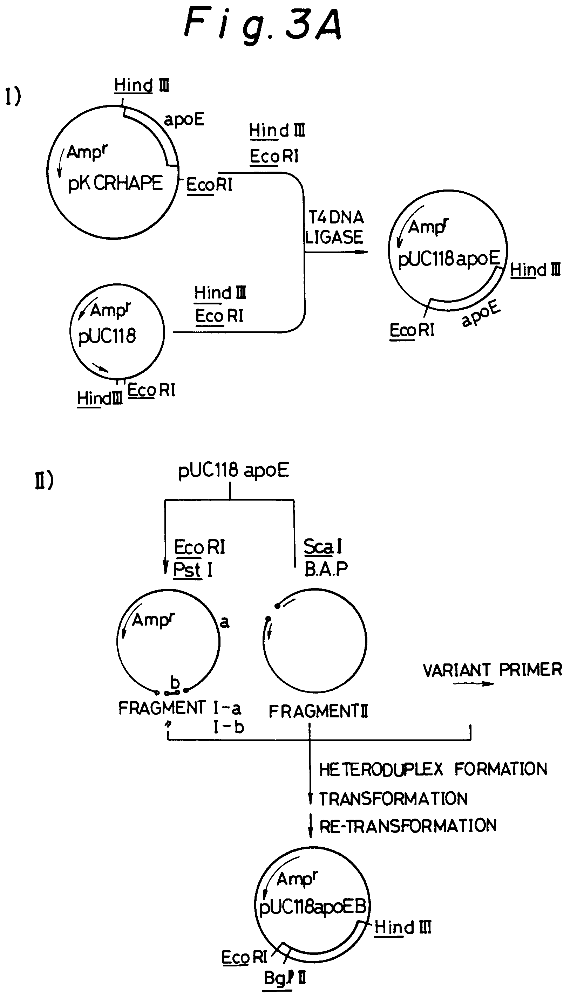

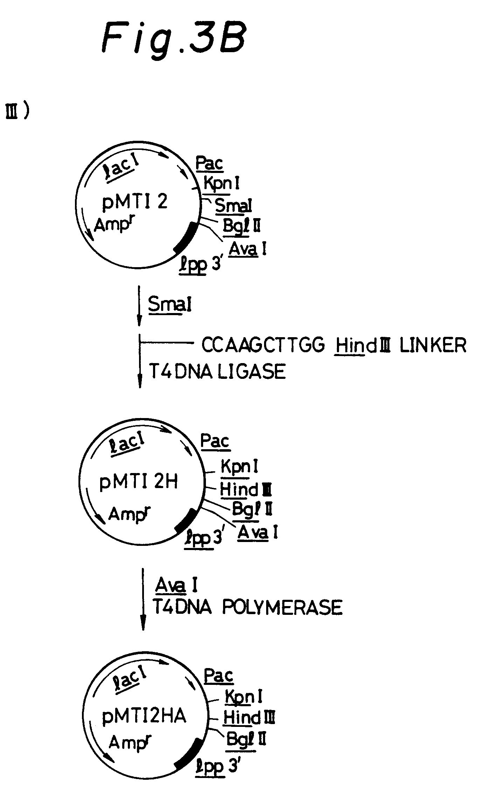

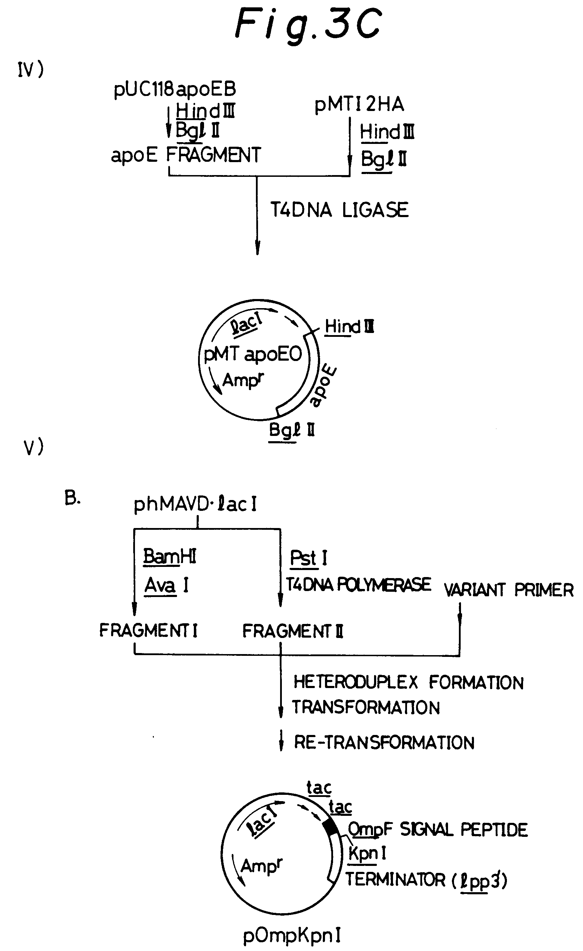

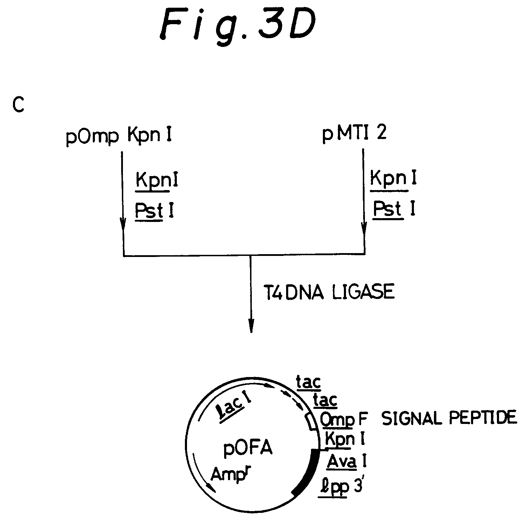

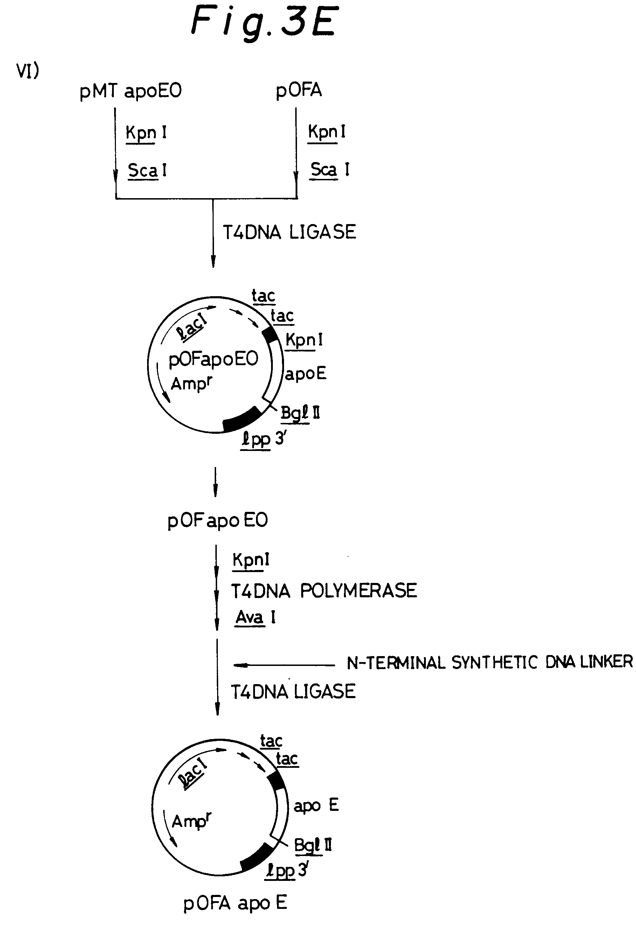

### EXAMPLE 1: Secretory Production in E. coli of Human Apolipoprotein E Using E. coli Outer Membrane Protein OmpF Signal Peptide (Fig. 3)

### I) Subcloning ApoE DNA of pKCRHAPE into pUC118

Two micrograms of pKCRHAPE, prepared in accordance with the procedures described in Japanese Patent Application Laying-open (KOKAI) No. 285990/1986, were completely digested with HindIII (2 u) in 20 µl of Buffer M (10 mM Tris-HCl, pH 7.5, 60 mM sodium chloride and 6 mM magnesium chloride) at 37°C for 2 hours. The reaction mixture was diluted with Buffer H (10 mM Tris-HCl, pH 7.5, 100 mM sodium chloride and 6 mM magnesium chloride). The total volume was adjusted to 40 µl. EcoRI (2 u) was added to the mixture and complete digestion was effected at 37°C for 2 hours. The reaction mixture was then heated at 70°C for 10 minutes to inactivate the enzyme. Thus, Liquid A was obtained.

Separately, pUC118 (2 µg) from TAKARA SHUZO, Japan was digested with HindIII and EcoRI under the conditions similar to those described above. The enzymes were inactivated to yield Liquid B.

Liquids A and B were mixed, dialyzed against water, and evaporated to dryness by a vacuum pump. Ten microliters of Buffer L (10 mM Tris-HCl, 6 mM magnesium chloride, 1 mM dithiothreitol and 1 mM ATP) were added to the mixture. T4 DNA ligase (1 u) was also added and reaction was effected at 4°C for 16 hours.

Five microliters of the resultant reaction mixture were used to transform commercially available JM109 competent cells (TAKARA SHUZO, Japan) according to the specifications. The resultant transformants were treated in conventional manners to isolate and purify plasmids. Thus, there was obtained plasmid pUC118apoE having the apoE DNA fragment inserted into pUC118.

### II) Introduction of BglII Cleavage Site into apoE Gene

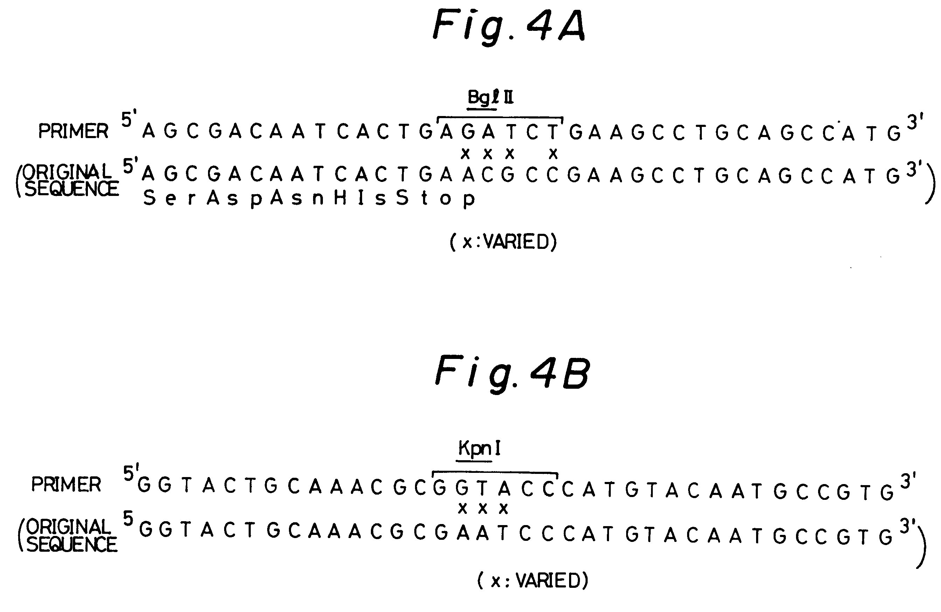

BglII cleavage site was introduced into the apoE gene in accordance with the procedures described in BIO/TECHNOLOGY, 636-639 (1984).
(1) The plasmid pUC118apoE (10 µg) was completely digested with EcoRI (10 u) and then PstI (10 u) in Buffer H (100 µl). In both cases, the reaction was effected at 37°C for 2 hours. The resulting reaction mixture was subjected to 5% polyacrylamide gel electrophoresis and stained with 0.05% ethidium bromide. Two larger DNA bands were cut from the gel and extracted in conventional manners. Thus, Fragments I-a and I-b were obtained.
(2) The plasmid pUC118apoE (1 µg) was completely digested with ScaI (1 u) in Buffer H (10 µl) at 37°C for 2 hours. Thereafter, alkaline phosphatase (2 u) derived from E. coli was added and reaction was effected at 65°C for one hour to remove 5′-terminal phosphate groups. The reaction mixture was treated with water-saturated phenol to remove proteins, and the phenol was removed by ether. The reaction mixture was dialyzed against water, evaporated to dryness by a vacuum pump, and then dissolved in water (10 µl). Thus, Fragment II was obtained.
(3) A variant primer (150 pmol) of 36 bases as shown in Fig. 4A was phosphorylated in the presence of T4 polynucleotide kinase (1 u) in Buffer K (50 mM Tris-HCl, pH 8.0, 10 mM magnesium chloride, 5 mM dithiothreitol and 10 mM ATP) at 37°C for one hour.
(4) A heteroduplex was formed in the following manner: 12 µl of 5X conc. polymerase ligase buffer (0.5 M sodium chloride, 32.5 mM Tris-HCl, pH 7.5, 40 mM magnesium chloride and 5 mM beta-mercaptoethanol) was mixed with Fragments I-a and I-b (each in 0.05 pmol), Fragment II (0.05 pmol) and the phosphorylated variant primer (45 pmol). The total volume was 34.5 µl. The resulting mixture was boiled at 100°C for 3 minutes, immediately after which it was placed in a thermostat at 30°C and allowed to stand for 30 minutes. Then, the mixture was allowed to stand at 4°C for 30 minutes and subsequently on ice for 10 minutes. Thus, the heteroduplex was formed.

To 11.6 µl of an aqueous solution containing the heteroduplex, there were added 2.5 mM 4-deoxynucleotide-3-phosphate, 10 mM ATP (2 µl), Klenow enzyme (2 u) and T4 DNA ligase (0.5 u). The total volume of this mixture was 20 µl. The reaction was effected at 12.5°C overnight to cyclize DNA.

Two microliters of an aqueous solution containing the cyclized DNA were used to transform E. coli JM109 by conventional procedures. Plasmids were isolated and purified from the transformants in conventional manners. The plasmids were cleaved with BglII restriction enzyme and subjected to 0.8% agarose gel electrophoresis to separate the cleaved plasmids (variant plasmids).

These variant plasmids were often found to be contaminated with original wild-type plasmids. These impure variant plasmids were again used to transform E. coli JM109. Thus, purified variant plasmid pUC118apoEB was obtained.

### III) Modification of Expression Vector

### A. Construction of Plasmid pMTI2 (Figs 5A and 5B)

(1) Preparation of Hybrid Promoter:
   Dna fragments having the same sequence as the -35 region of T5 P25 promoter (DNA Fragments 1 and 2 as shown below) and DNA fragments having the same sequence as the -10 region of the promoter, the operator region and the transcription initiation site of lacUV5 (DNA Fragments 3 and 4 as shown below) were prepared by a DNA synthesizer Model 308A manufactured by NIKKAKI, Japan. 
1. 5'AATTCAAAAATTTATTTGCTTTCAGGAAAATTTTTCT3'

2. 5'ATACAGAAAAATTTTCCTGAAAGCAAATAAATTTTTG3'

3. 5'GTATAATGTGTGGAATTGTGAGCGGATAACAATTTCGGATCCA3'

4. 5'AGCTTGGATCCGAAATTGTTATCCGCTCACAATTCCACACATT3'

EcoRI -35
1 5'AATTCAAAAATTTATTTGCTTTCAGGAAAATTTTTCT3'
3'GTTTTTAAATAAACGAAAGTCCTTTTAAAAAGACATA5' 2

-10 Operator BamHI
3. 5'GTATAATGTGTGGAATTGTGAGCGGATAACAATTTCGGATCCA3'
3'TTACACACCTTAACACTCGCCTATTGTTAAAGCCTAGGTTCGA5' 4
HindIII
   1: 37 bases which correspond to the DNA fragment containing the -35 region of P25 promoter of T5 phage;
   2: 37 bases which is a DNA fragment complementary to 1;
   3: 43 bases which correspond to the DNA fragment containing the -10 and operator regions of lacUV5; and
   4: 43 bases which is a DNA fragment complementary to 3.

   Each (100 µg) of the synthetic DNA fragments was deprotected in 28% aqueous ammonia solution (2 ml) at 55°C for 6 hours and purified on 17% acrylamide/7 M urea gel to yield each fragment (10 µg). Each DNA Fragment (1 µg) was treated with T4 polynucleotide kinase (1 u) in 10 µl of a buffer containing 10 mM Tris-HCl (pH 7.6) and 10 mM magnesium chloride at 37°C for one hour to phosphorylate the 5′-terminal.
   In 50 mM sodium chloride solution, DNA Fragment 1 was mixed with DNA Fragment 2 while DNA Fragment 3 was separately mixed with DNA Fragment 4. After heating at 100°C for 3 minutes, each mixture was gradually cooled to yield a double-stranded 37 bp or 43 bp DNA fragment, each in an amount of 2 µg.
   These DNA fragments (each in 0.5 µg) and plasmid pBR322 (1 µg) cleaved with EcoRI and HindIII were treated with T4 DNA ligase (1 u) in 10 µl of a buffer containing 10 mM Tris-HCl (pH 7.6). 6 mM magnesium chloride, 10 mM ATP and 1 mM dithiothreitol at 4°C for 16 hours.
   To this reaction mixture, there was added calcium chloride to a final concentration of 100 mM. The mixture was mixed with E. coli HB101 competent cells and treated at 0°C for 10 minutes and then at 37°C for 5 minutes. The resulting transformants could be readily selected in a medium containing 20 µg/ml tetracycline since the desired transformant had acquired tetracycline resistance.
   Thus, pac promoter plasmid pBRpac having promoter activities was obtained. The base sequence of the pac promoter was determined by the conventional dideoxy method, which is fully described in Anal. Biochem., 152, 232 (1986).
   The competent cells used herein were obtained by collecting cells in the logarithmic growth phase by conventional procedures, suspending the cells in 1/3 volume of 0.1 M calcium chloride solution at 0°C, allowing to stand for 30 minutes, collecting again the cells, and suspending them in 1/100 volume of 0.1 M calcium chloride solution.
(2) Introduction of Transcription Termination Sequence into Hybrid Promoter:
   One microgram of the plasmid pBRpac (4.4 kbp) was digested with BamHI (1 u) and PvuII (1 u) in Buffer H (10 µl) at 37°C for 2 hours. The product was precipitated with ethanol, evaporated to dryness and stored.
   On the other hand, 5 µg of plasmid pINIIIA1 (The EMBO J., Vol. 3, No. 10, 2437 (1984)) was digested with KpnI (5 u) in 50 µl of a buffer containing 10 mM Tris-HCl and 6 mM magnesium chloride at 37°C for 2 hours. The reaction mixture was mixed with 5.5 µl of a buffer containing 330 mM Tris-HCl (pH 7.9), 660 mM potassium acetate and 100 mM magnesium acetate, treated with T4 DNA polymerase (10 u) at 37°C for 15 minutes to remove the protruding 3′-terminal, and heated at 70°C for 10 minutes to inactivate the enzyme. The reaction mixture was mixed with 6.0 µl of a buffer containing 100 mM Tris-HCl (pH 7.5), 1 M sodium chloride and 60 mM magensium chloride and treated with BamHI (5 u) at 37°C for 2 hours. After removal of proteins by addition of water-saturated phenol (60 µl), ethanol precipitation was repeated three times to purify DNA. Thus, there was obtained 0.5µg of an about 500 bp fragment of lpp 3′-region containing the transcription termination sequence.
   This DNA fragment and the above digested plasmid pBRpac were treated with T4 DNA ligase (1 u) in 10 µl of a buffer containing 10 mM Tris-HCl (pH 7.5), 10 mM magnesium chloride, 10 mM ATP and 1 mM dithiothreitol at 4°C for 16 hours. Thus, plasmid pPac was obtained.
   This plasmid pPac could be stably maintained in E. coli lacI^{q} strain JM105 or JM109. The plasmid pPac (0.1 µg) was used to transform E. coli strain JM105 according to the method described in (1) above. The resulting transformant was propagated in a minimal medium (1 liter) containing 20 mg/ml ampicillin. The plasmid was isolated from the transformant and purified by conventional procedures. Thus, the plasmid pPac was obtained in an amount of 1 mg.
(3) Introduction of Multi-Linker:
   A multi-linker comprising the SD sequence and the initiation Met codon as shown in Fig. 5B was synthesized in Model 308A synthesizer (NIKKAKI, Japan) and purified according to the method described in (1) above. Thus, 10 µg of DNA was obtained.
   On the other hand, the plasmid pPac (1 µg) was cleaved with BamHI (1 u) in 10 µl of a buffer containing 10 mM Tri-HCl (pH 7.5), 100 mM sodium chloride and 6 mM magnesium chloride at 37°C for 2 hours, precipitated with ethanol, evaporated to dryness and stored.
   The cleaved plasmid pPac (1 µg) and the multi-linker (1 µg) were treated with T4 DNA polymerase (1 u) in 10 µl of a buffer containing 10 mM Tris-HCl (pH 7.5), 10 mM magnesium chloride, 10 mM ATP and 1 mM dithiothreitol at 4°C for 16 hours. Thus, plasmid pMT2 into which the multi-linker was inserted was obtained.
(4) Introduction of lac Repressor Gene:
   Ten micrograms of plasmid pMC9 (Proc. Natl. Acad. Sci. USA, 80, 3015 (1983)) was cleaved with EcoRI (10 u) in 100 µl of a buffer containing 100 mM Tris-HCl (pH 7.5), 10 mM sodium chloride and 6 mM magnesium chloride at 37°C for 2 hours, precipitated with ethanol, evaporated to dryness, and stored. Thus, 1.7 kbp DNA fragment coding for lacI repressor gene was obtained in an amout of 2.5 µg.

The plasmid pMT2 (1 µg) was cleaved with EcoRI (1 u) in 10 µl of a buffer containing 100 mM Tris-HCl (pH 7.5), 10 mM sodium chloride and 6 mM magnesium chloride at 37°C for 2 hours, precipitated with ethanol, evaporated to dryness and stored.

This cleaved plasmid pMT2 (1 µg) and the 1.7 kbp DNA fragment (1 µg) were ligated by the treatment with T4 DNA ligase (1 µg) in 20 µl of a buffer containing 10 mM Tris-HCl (pH 7.5), 10 mM magnesium chloride, 10 mM ATP and 1 mM dithiothreitol to yield plasmid pMTI2.

### B. Modification

One microgram of the plasmid pMTI2 prepared in A above was digested with SmaI (1 u) in 10 µl of Buffer S (10 mM Tris-HCl, pH 7.5, 6 mM potassium chloride and 6 mM magnesium chloride) at 37°C for 2 hours. The reaction mixture was heated at 70°C for 10 minutes to inactivate the enzyme, dialyzed against water, and evaporated to dryness. The reaction mixture was mixed with about 50 pmol of a HindIII linker phosphorylated at 5′-terminal (5′pCCAAGCTTGG, TAKARA SHUZO, Japan), and treated with T4 DNA ligase (1 u) in Buffer L (10 µl) at 4°C for 16 hours. The resulting reaction mixture was used to transform E. coli JM109 competent cells. DNA was isolated and purified from the transformant, and cut with HindIII. Thus, plasmid pMTI2H having the linker DNA inserted thereinto was obtained.

The plasmid pMTI2H (1 µg) was digested with AvaI (1 u) in Buffer M (10 µl) at 37°C for 2 hours and heated at 70°C for 10 minutes to inactivate the enzyme. The reaction mixture was dialyzed against water and evaporated to dryness. The mixture was then treated with T4 DNA polymerase (1 u) in 20 µl of Buffer P (33 mM Tris-acetic acid, pH 7.9, 66 mM potassium acetate 10 mM magnesium acetate and 2.5 mM 4-deoxynucleotide triphosphate) at 37°C for 15 minutes. The enzyme was inactivated by heat treatment at 70°C for 10 minutes. The resulting mixture was dialyzed against water and evaporated to dryness. The mixture was treated with T4 DNA ligase (1 u) in Buffer L (10 µl) at 4°C for 16 hours. The resulting mixture was used to transform E. coli JM109 competent cells. DNA was isolated and purified from the transformants and digested with AvaI. Thus, there was obtained plasmid pMTI2HA from which the AvaI cleavage site had been deleted.

### IV) Introduction of ApoE DNA Fragment into pMTI2HA

Ten micrograms of pUC118apoEB were digested with HindIII (20 u) and BglII (20 u) in Buffer M (100 µl) at 37°C for 2 hours. Approximately 1 kbp DNA fragment (apoE fragment) containing the gene coding for apoE was separated by 5% acrylamide gel electrophoresis, stained with 0.05% ethidium bromide, excised from the gel, and extracted in conventional manners.

On the other hand, pMTI2HA (1 µg) was digested with HindIII (1 u) and BglII (1 u) in Buffer M (10 µl) at 37°C for 2 hours, and treated with water-saturated phenol to precipitate proteins. After removing the phenol by addition of ether, the product was dialyzed against water and evaporated to dryness.

The plasmid DNA was mixed with the apoE fragment (1 pmol) and the resulting mixture was treated with T4 DNA ligase (1 u) in Buffer L (10 µl) at 4°C for 16 hours. The reaction mixture was used to transform E. coli JM109 competent cells. Plasmids were isolated and purified from the transformants and investigated by digestion with HindIII and BglII. Thus, plasmid pMTapoEO having the apoE fragment inserted thereinto was obtained.

### V) Construction of Secretory Vector

### A. Construction of Plasmid phMAVD lacI (Fig. 6): Japanese Patent Application Laying-open KOKAI No. 107996/1988

(1) Human heart sections were broken in liquid nitrogen, and homogenized in an aqueous guanidinium thiocyanate solution. The resulting homogenate was subjected to cesium chloride equilibrium density gradient ultracentrifugation according to the method described by Chirgwin et al. in Biochemistry, 18, 5294-5299 (1979) to separate the whole RNA. The whole RNA was purified by oligo(dT) cellulose column chromatography in conventional manners. The isolated poly(A)-containing RNA was used as mRNA materials in the following steps.
(2) A vector primer and an oligo(dG)-tailed linker were prepared from a hybrid plasmid of pBR322 and SV40 in accordance with the procedures described by Okayama and Berg in Molecular and Cellular Biology, 2, 161-170 (1982):
   The hybrid plasmid (400 µg) of pBR322 and SV40 (0.71-0.86 in map unit) was digested with KpnI restriction enzyme in a buffer containing bovine serum albumin at 37°C for 4 hours. DNA was collected by conventional ethanol precipitation and dissolved in a dTTP-containing buffer. The solution was incubated at 37°C for 30 minutes in the presence of terminal deoxynucleotidyl transferase to add an about 60 dT tail to the cleaved KpnI site. The tailed DNA was collected by ethanol precipitation and then digested with HpaI restriction enzyme in a buffer containing bovine serum albumin at 37°C for 5 hours. The resulting larger DNA fragment was purified by agarose gel electrophoresis and collected by the glass powder procedure: Vogelstein et al., Proc. Natl. Acad. Sci. U.S.A., 76, 615-619 (1979). The DNA was added to an oligo(dA) cellulose column at 0°C, eluted with water, and collected by ethanol. Thus, a vector primer having an oligo(dT) tail was obtained.
   On the other hand, 100 µg of the hybrid plasmid of pBR322 and SV40 (0.19-0.32 in map unit) was digested with PstI restriction enzyme in a buffer containing bovine serum albumin at 37°C for 1.5 hours. DNA was collected and dissolved in a dGTP-containing buffer. The solution was incubated at 37°C for 20 minutes in the presence of terminal deoxynucleotidyl transferase to add an about 10-15 dG tail. The collected DNA was digested with HindIII restriction enzyme in a buffer containing bovine serum albumin at 37°C for one hour and subjected to 1.8% agarose gel electrophoresis. Thus, a small oligo(dG)-tailed linker DNA was extracted and collected.
(3) A cDNA library was prepared by the procedures described by Okayama and Berg in Molecular and Cellular Biology, 2, 161-170 (1982):
   The mRNA materials (30 µg) prepared in (1) above and the vector primer (10 µg) prepared in (2) above were added to an aqueous solution containing Tris-HCl (pH 8.3). magnesium chloride, potassium chloride, dithiothreitol, dATP, dTTP. dGTP and ³²P-dCTP. The mixture was incubated at 37°C for 20 minutes in the presence of reverse transcriptase to synthesize plasmid-cDNA:mRNAs. These materials were collected in the form of pellet by ethanol precipitation. The collected pellets were dissolved in a buffer containing cobalt chloride, dithiothreitol, poly(A), ³²P-dCTP and terminal deoxynucleotidyl transferase and incubated at 37°C for 10 minutes to add 10 to 15 dCMP residues to each end.
   Then, the collected pellets containing oligo(dC)-tailed plasmid-cDNA:mRNA were dissolved in a buffer containing bovine serum albumin and digested with HindIII restriction enzyme at 37°C for one hour. Thus, HindIII-digested oligo(dC)-tailed cDNA:mRNA plasmids were collected by ethanol precipitation.
   The HindIII-digested plasmids were dissolved in a buffer containing the oligo(dG)-tailed linker DNA prepared in (2) above, incubated at 65°C for 2 minutes, allowed to stand at 42°C for 30 minutes, and then cooled to 0°C. The reaction mixture was mixed with E. coli DNA ligase and incubated overnight in the presence of beta-NAD (nicotinamide adenine dinucleotide). Then, dATP, dTTP, dGTP, dCTP, beta-NAD, E. coli DNA ligase, E. coli DNA polymerase and E. coli RNase H were added and the resulting mixture was incubated at 12°C for one hour and then at room temperature for one hour. The mixture was then cooled to terminate the reaction. Thus, desired cDNA fragment-containing plasmids were obtained.
   These plasmids were used to transform E. coli HB101 by conventional procedures. In these manners, a cDNA library was established.
(4) Two groups of oligonucleotides which are complementary to the DNA sequence coding for Met-Asp-Arg-Ile-Gly of cardionatrin were synthesized: 
Oligo I                    3'-TAC CTA GCA TAA CC-5'
                                     G   G
                                     C   T
                                     T
                                   TCT
                                     C

Oligo II                   3'-TAC CTG GCA TAA CC-5'
                                     G   G
                                     C   T
                                     T
                                   TCT
                                     C
 Using these Oligos I and II as probes, the cDNA library was screened. Twelve (12) clones hybridizing with the probes were selected from 40,000 transformants. From these 12 clones, cDNA fragments were extracted and their restriction enzyme maps were established. Maxam-Gilbert method described in Methods in Enzymology, 65, 499-560 (1980) was used to determine base sequences of the cDNA fragments. One of them was designated as pHANF48: Nature, 310, 23, 699 (1985).
(5) The pHANF48 described in (4) above was digested with PvuII and RsaI to isolate a 301 bp fragment comprising AVD DNA. This fragment was ligated to a methionine linker by the treatment with T4 DNA ligase at 8°C for 14 hours and digested with HindIII restriction enzyme at 37°C for 2 hours to yield a 333 bp fragment. This fragment was ligated to a linker containing a ribosome binding sequence by the treatment with T4 DNA ligase at 8°C for 14 hours and digested with BamHI at 37°C for 2 hours to yield a 365 bp fragment.
   Plasmid pUC8 (TAKAKA SHUZO, Japan) was digested with BamHI restriction enzyme at 37°C for 2 hours. The 365 pb fragment was inserted into the BamHI site of pUC8. The resulting plasmid was used to transform E. coli JM105. White colonies on Xgal were selected. Thus, plasmid pUCcd8 was obtained.
   The plasmid pUCcd8 was partially digested with AvaI restriction enzyme at 37°C for 30 minutes and ligated to the following linker containing a stop codon (termination linker) in the presence of T4 DNA polymerase to yield plasmid pUCcd8term. 
Termination Linker: TGA TAG
ACT ATC
 This plasmid pUCcd8term was digested with ApaI and BamHI at 37°C for 2 hours to yield a 260 bp fragment. On the other hand, plasmid pDR540 (Gene, 20, 231 (1982)) having tac promoter was digested with restriction enzymes EcoRI and BamHI at 37°C for 2 hours to yield an about 370 bp fragment. Further, pHANF48 described in (4) above was digested with ApaI and EcoRI to yield a large fragment containing the ampicillin resistance gene.
   These three fragments were ligated to each other by the treatment with T4 DNA ligase at 8°C for 14 hours. The resulting plasmid was used to transform E. coli JM105. Thus, plasmid phAVD was obtained from the transformant.
(6) Plasmid pHF006 as described in Nucleic Acid Research, 10, 6957 (1982) was digested with PstI restriction enzyme to yield a 178 bp PstI fragment.On the other hand, plasmid pSP64 available from Boehringer Manheim was digested with PstI restriction enzyme and treated with alkaline phosphatase. This digested plasmid was then ligated to the 178 bp PstI fragment. Thus, plasmid pSP64.OmpF was obtained.
   This plasmid pSP64.OmpF was digested with HindIII restriction enzyme and treated with Bal31. The resulting plasmid DNA was further treated with T4 DNA polymerase to form blunt ends. HindIII linker was ligated to the treated plasmid DNA, digested with HindIII and cyclized in the presence of ligase. The resulting plasmid was used to transform E. coli. Plasmid DNA was separated from the transformant.
   This plasmid DNA was digested with HindIII and PstI. A small fragment of less than 150 bp was selected (clone No. 33). The PstI-HindIII fragment was 130 bp. The selected plasmid pSP64.OmpF No. 33 was digested with PstI and treated with T4 DNA polymerase to form blunt ends. HpaI linker was ligated to the blunt end, digested with HpaI and cyclized by ligase. Thus, plasmid pSP64.OmpF.HpaI was obtained.
(7) The plasmid pUC8 as described in (5) above was digested with EcoRI and then HindIII to yield an EcoRI-HindIII fragment containing the Amp^{r} gene.
   The plasmid pSP64.OmpF.HpaI obtained in (6) above was digested with EcoRI and then HindIII to yield an about 200 bp fragment. This fragment was further digested with HhaI. Thus, an 85 bp fragment containing the OmpF signal peptide region was obtained. 
A synthetic linker:

HindIII                HhaI
AAGCTTATGATGAAGCG
      ATACTACTTC
 was ligated to the two fragments in the presence of T4 DNA ligase. Thus, plasmid pUC.OmpF.S+Met was obtained.
(8) The plasmid pUC.OmpF.S+met was digested with HindIII and FnuDII to yield an about 70 bp fragment containing the OmpF signal peptide region.

On the other hand, the plasmid pHANF48 as described in (4) above was digested with AluI to yield a 269 bp fragment containing the AVD gene region. This fragment was further digested with ApaI to yield an about 220 bp fragment.

These two fragments were ligated to each other and further to a larger fragment obtained by digesting the above described plasmid PhAVD with HindIII and ApaI, in the presence of T4 DNA ligase. Thus, plasmid phAVD.delta.ST was obtained.

A larger fragment obtained by digesting the plasmid phAVD.delta.ST with AvaI was ligated to a smaller fragment obtained by digesting the plasmid phAVD with AvaI, in the presence of T4 DNA ligase. Thus, plasmid phMAVD.delta.P was obtained.

This plasmid phMAVD.delta.P was digested with HindIII and treated with BAP. A 104 bp tac promoter obtained by digesting the phAVD with HindIII was inserted into the BAP-treated plasmid. Thus, plasmid phMAVD was obtained.

This plasmid phMAVD was digested with EcoRI and treated with BAP. On the other hand, plasmid pMC9 was digested with EcoRI to yield a 1.7 kbp fragment containing the lacI gene. This fragment was ligated to the BAP-treated plasmid. Thus, plasmid phMAVD.lacI as shown in Fig. 6 was obtained.

### B. Construction of Plasmid pOmpKpni (Fig. 3C)

(1) The plasmid phMAVD.lacI (10 µg) constructed in A above was digested with BamHI (10 u) and AvaI (10 u) in Buffer H (100 µl) at 37°C for 2 hours. DNA fragments were separated by 5% acrylamide gel electrophoresis and stained with 0.05% ethidium bromide. A large DNA fragment (Fragment I) was cut from the gel and extracted in conventional manners.
(2) The plasmid phMAVD.lacI (1 µg) was digested with PstI (1 u) in Buffer H (10 µl). Then, the reaction mixture was heated at 70°C for 10 minutes to inactivate the enzyme. The reaction mixture was dialyzed against water and evaporated to dryness.
   The dried material was treated with T4 DNA polymerase in Buffer P (20 µl) at 37°C for 15 minutes. The reaction mixture was heated at 70°C for 10 minutes to inactivate the enzyme, dialyzed against water, evaporated to dryness, and dissolved in water (10 µl). Thus, an aqueous solution of Fragment II was obtained.
(3) A variant primer (150 pmol) of 36 bases as shown in Fig. 4B was phosphorylated in the presence of T4 polynucleotide kinase (1 u) in Buffer K (10 µl) at 37°C for one hour.
(4) A heteroduplex was formed in the following manner: 12 µl of 5X conc. polymerase-ligase buffer (0.5 M sodium chloride, 32.5 mM Tris-HCl, pH 7.5, 40 mM magnesium chloride and 5 mM beta-mercaptoethanol) was mixed with Fragment I 0.05 pmol), Fragment II (0.05 pmol) and the 5′-phosphorylated primer (45 pmol). The total volume was adjusted to 34.8 µl. The resulting mixture was boiled at 100°C for 3 minutes, immediately after which it was placed in a thermostat at 30°C and allowed to stand for 30 minutes. Then, the mixture was allowed to stand at 4°C for 30 minutes and subsequently on ice for 10 minutes. Thus, the heteroduplex was formed.
(5) To 11.6 µl of an aqueous solution containing the heteroduplex, there were added 2.5 mM 4-deoxynucleotide triphosphate (2 µl), 10 mM ATP (2 µl), Klenow enzyme (2 u) and T4 DNA ligase (0.5 u). The total volume of this mixture was adjusted to 20 µl. The reaction was effected at 12.5°C for 16 hours to cyclize DNA.

Two microliters of an aqueous solution containing the cyclic DNA were used to transform E. coli JM109 by conventional procedures. Plasmids were isolated and purified from the transformants in conventional manners. The plasmids were cleaved with KpnI restriction enzyme and subjected to 0.8% agarose gel electrophoresis to separate the cleaved plasmids (variant plasmids).

These variant plasmids were often found to be contaminated with original wild-type plasmids. These impure variant plasmids were again used to transform E. coli JM109. Thus, purified variant plasmid pOmpKpnI was obtained.

### C. Construction of Plasmid pOFA (Fig. 3D)

One microgram of pMTI2 was digested with KpnI (1 u) in Buffer L (10 µl) at 37°C for 2 hours. The reaction mixture was then heatd at 70°C for 10 minutes to inactivate the enzyme, dialyzed against water and evaporated to dryness. The plasmid DNA was digested with PstI (1 u) in Buffer H (10 µl) at 37°C for 2 hours. The reaction mixture was then heated at 70°C for 10 minutes to inactivate the enzyme, dialyzed against water and evaporated to dryness.

On the other hand, pOmpKpni was digested with KpnI and PstI by similar procedures. The resulting plasmid DNA was evaporated to dryness.

These digested DNA fragments were dissolved in Buffer L (10 µl) and treated with T4 DNA ligase (1 u) at 4°C for 16 hours. The reaction mixture (3 µl) was used to transform E. coli JM109 competent cells. Plasmids were isolated and purified from the transformants, and cut with HindIII and/or SmaI. Thus, there was obtained plasmid pOFA in which the fragment containing pOmpKpni lacI-OmpF was ligated to the fragment containing pMTI2 replication origin, lpp transcription termination sequence and the multi-cloning sequence.

### D. Construction of Plasmid pOFAapoE (Fig. 3E)

One microgram of pMTapoEO (Fig. 3C) was digested with KpnI (1 u) in Buffer L (10 µl) at 37°C for 2 hours. The reaction mixture was then heated at 70°C for 10 minutes to inactivate the enzyme, dialyzed against water and evaporated to dryness. The plasmid DNA was then digested with ScaI (1 u) in Buffer H (10 µl) at 37 C for 2 hours. Proteins were extracted with water-saturated phenol and the phenol was extracted with ether. The resulting mixture was dialyzed against water and evaporated to dryness.

On the other hand, POFA (1 µg) was digested with KpnI and ScaI by similar procedures and the resulting plasmid DNA was evaporated to dryness.

These digested DNA fragments were ligated to each other in Buffer L (10 µl) in the presence of T4 DNA ligase (1 u) at 4°C for 16 hours. The reaction mixture (2 µl) was used to transform E. coli JM109 competent cells. Plasmids were isolated and purified from the transformants. The purified DNAs were checked by digestion with EcoRV and 0.8% agarose gel electrophoresis. Thus, there was obtained plasmid pOFapoEO in which the fragment containing pOFA lacI-OmpF was ligated to the fragment containing pMTapoEO replication origin and apoE gene.

The plasmid pOFapoEO (1 µg) was digested with KpnI (1 u) in Buffer L (10 µl) at 37°C for 2 hours. The reaction mixture was heated at 70°C for 1.0 minutes to inactivate the enzyme, dialyzed against water and evaporated to dryness. Then, the mixture was treated with T4 DNA polymerase (1 u) in Buffer P (20 µl) at 37°C for 15 minutes to remove the protruding 3′-terminal produced by the KpnI digestion. Thus, a blunt end was produced at 3′-terminal of the plasmic DNA. The reaction mixture was then heated at 70°C for 15 minutes to inactivate the enzyme, dialyzed against water and evaporated to dryness. The resulting DNA fragment was digested with AvaI (1 u) in Buffer M (10 µl) at 37°C for 2 hours. The reaction mixture was then treated with water-saturated phenol to remove proteins, treated with ether to remove the phenol, dialyzed against water and evaporated to dryness to yield a DNA fragment (Fragment A).

The following DNA linker corresponding to the N-terminal of apoE was synthesized by a DNA synthesizer (Applied Biosystem M380 manufactured by NIKKAKI, Japan) and purified in conventional procedures: 
Lys Val Glu Gln Ala Val Glu Thr Glu Pro Glu AvaI
AAA GTA GAA CAA GCT GTA GAA ACA GAA CCA GAA C
TTT CAT CTT GTT CGA CAT CTT TGT CTT GGT CTT GGG CT
This synthetic DNA linker (150 pmol) was phosphorylated at 5′-terminal by the treatment with T4 DNA polynucleotide kinase (1 u) in Buffer K (10 µl). The reaction mixture was mixed with 200 mM sodium chloride (10 µl), boiled at 100°C for 3 minutes, and gradually annealed.

The 5′-phosphorylated DNA linker (15 pmol) and Fragment A prepared above were mixed together and ligated to each other by the treatment with T4 DNA ligase (1 u) in Buffer L (10 ul) at 4°C for 16 hours. The resulting mixture was used to transform E. coli JM109 competent cells. Plasmids were isolated and purified from the transformants. The purified plasmid DNA was checked by digestion with BamHI and AvaI and 5% acrylamide gel electrophoresis. Thus, desired plasmid pOFAapoE was obtained.

### VII) Secretory Production of Apolipoprotein E

E. coli JM109 containing pOFAapoE was cultivated in L broth (10 g bactotrypton, 5 g bactoyeast extract, 10 g sodium chloride, 20 mg ampicillin, in 1 liter water) at 30°C overnight. The culture was transferred to 50 volumes of new L broth and cultivated at 30°C for 5 hours. Isopropyl-beta-Dthiogalactopyranoside, which is an inducer of the tac promoter on the plasmid, was added in a concentration of 2 mM to induce secretory production of apoE. Cultivation was further conducted for 3 hours.

The culture was centrifuged at 6,000 r.p.m. for 10 minutes to collect cells. The wet cells (1 g) were suspended in 20 ml of a solution containing 33 mM Tris-HCl (pH 8.0) and 20% sucrose and shaked at 25°C for 10 minutes. The cells were collected by centrifugation at 10,000 r.p.m. for 10 minutes. After discarding the supernatant, cool water (20 ml) was added to the cells and the mixture was thoroughly stirred in ice water for 10 minutes. The cells were removed by centrifugation at 8,000 r.p.m. for 10 minutes. The supernatant (periplasmic fraction) was lyophilized and electrophoresed on SDS polyacrylamide gel. It was thus shown that large quantities of human apolipoprotein E was produced and secreted into the periplasm. It was also found that the N-terminal of this human apolipoprotein E was natural-type Lys-Val-Glu-Gln-Ala-Val.

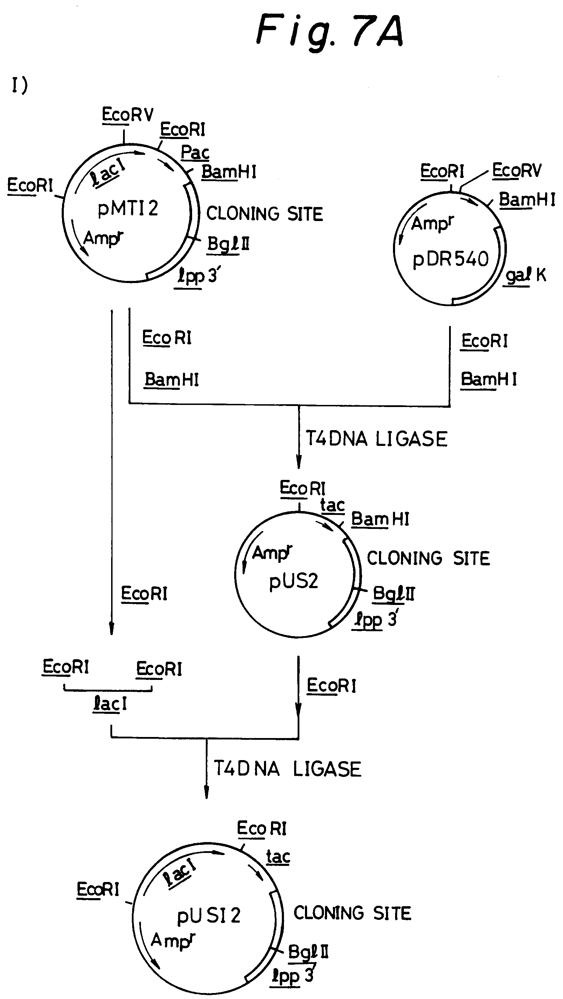

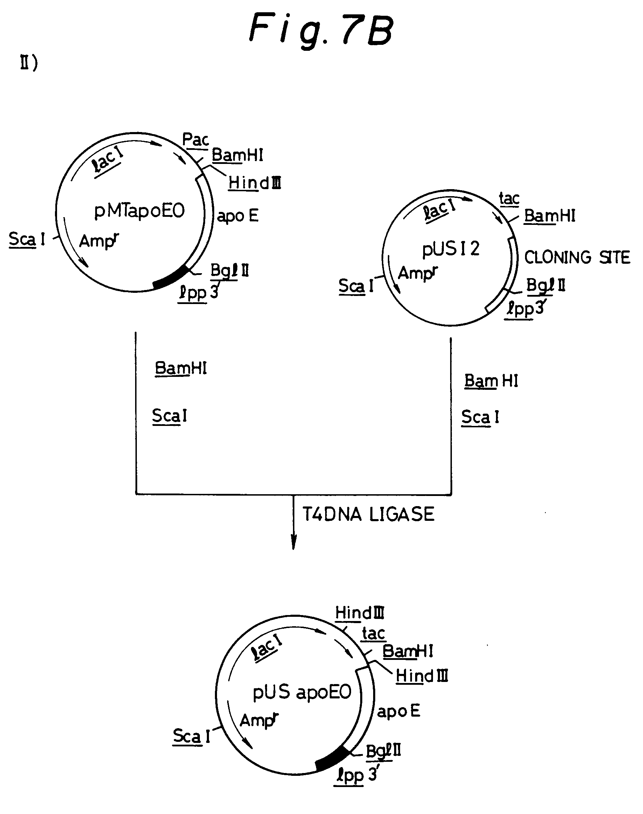

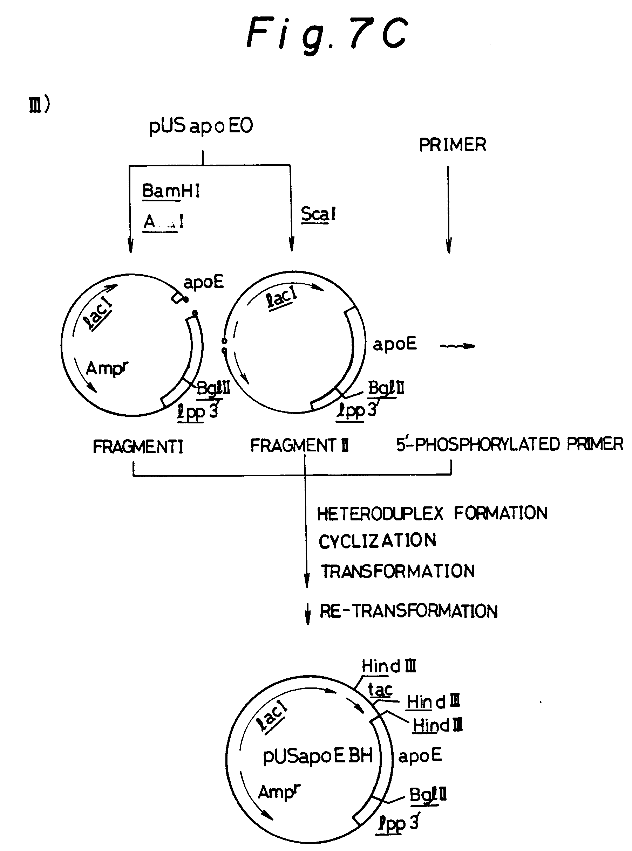

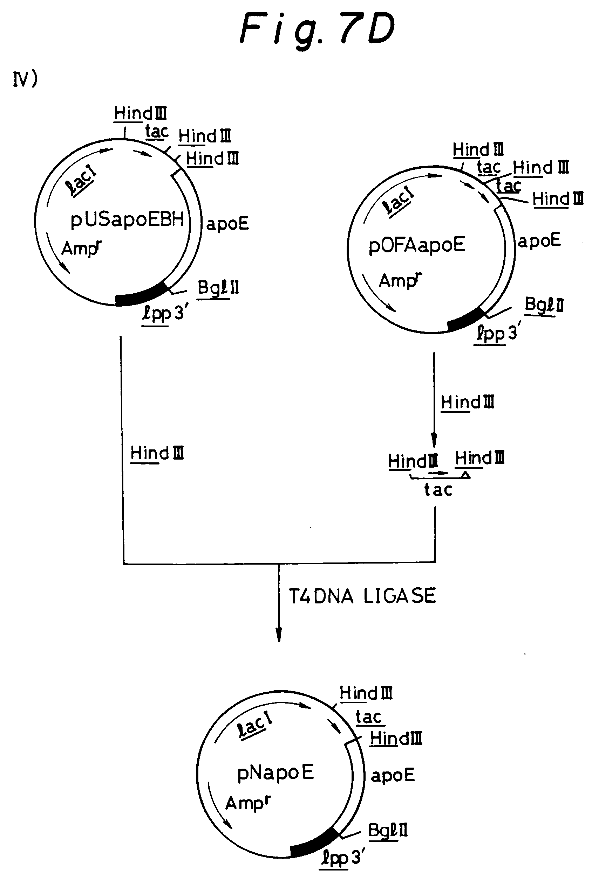

### EXAMPLE 2: Secretory Production in E. coli of Human Apolipoprotein E Using Signal Peptide of Human Apolipoprotein E (Fig. 7)

### I) Construction of tac Promoter Vector pUSI2 (Fig. 7B)

The plasmid pMTI2 (1 µg) as shown in Fig. 5B was digested with EcoRI (1 u) and BamHI (1 u) in Buffer H (10 µl) at 37°C for 2 hours. The reaction mixture was heated at 70°C for 10 minutes to inactivate the enzymes.

On the other hand, pDR540 (1 µg) purchased from Farmacia was digested with EcoRI and BamHI by similar procedures.

These reaction mixtures were mixed and treated with T4 DNA ligase (1 u) in 10X Buffer L (2 µl) at 4°C for 16 hours to ligate the DNA fragments to each other. The ligation mixture (10 µl) was used to transform E. coli JM109 competent cells. Plasmids were isolated and purified from the transformants. The plasmid DNAs were checked by digestion with EcoRV and 0.8% agarose gel electrophoresis. Thus, there was obtained plasmid pUS2 into which the tac promoter fragment of pDR540 was inserted.

This plasmid pUS2 lacked the lacI-containing EcoRI fragment as a result of the EcoRI digestion. The plasmid pMTI2 was digested with EcoRI and the 1.7 kbp lacI-containing EcoRI fragment was isolated and purified by conventional acrylamide gel electrophoresis. The purified fragment was introduced into the EcoRI site of pUS2. The resulting plasmid was designated as pUSI2.

### II) Construction of Plasmid pUSapoEO (Fig. 7B)

The plasmid pMTapoEO (1 µg) as shown in Fig. 3C was digested with BamHI (1 u) and ScaI (1 u) in Buffer H (10 µl) at 37°C for 2 hours. The reaction mixture was heated at 70°C for 10 minutes to inactivate the enzymes.

On the other hand, pUSI2 (1 µg) constructed in I) above was digested with ScaI and BamHI by similar procedures.

These reaction mixtures were mixed and treated with T4 DNA ligase (1 u) in 10X conc. Buffer L (2 µl) at 4°C for 16 hours to ligate the DNA fragments to each other. The ligation mixture (10 µl) was used to transform E. coli JM109 competent cells. Plasmids were isolated and purified from the transformants. The plasmid DNAs were checked by digestion with EcoRV and 0.8% agarose gel electrophoresis. Thus, there was obtained plasmid pUSapoEO into which the tac promoter fragment of pUSI2 was inserted.

### III) Introduction of HindIII Site into N terminal of Human Apolipoprotein E Signal Peptide by Site-Directed Mutagenesis

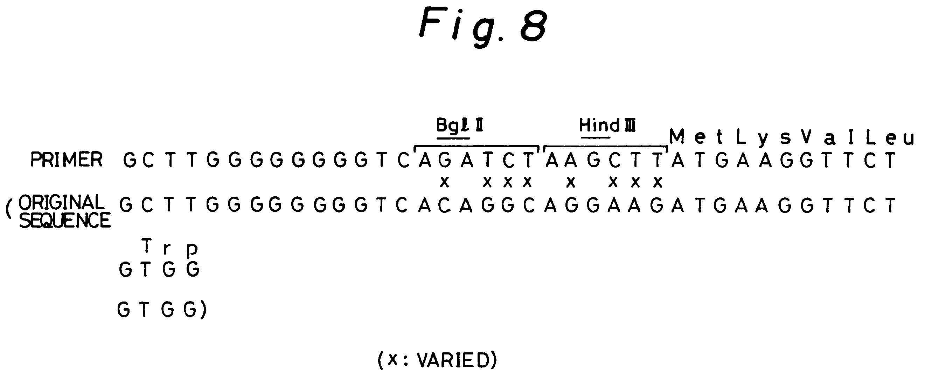

(1) The plasmid pUSapoEO (10 µg) was digested with BamHI (10 u) and AvaI (10 u) in Buffer H (100 µl) at 37°C for 2 hours. The digestion mixture was electrophoresed on 5% acrylamide gel and stained with 0.05% ethidium bromide. The larger DNA fragment (Fragment I) was cut from the gel and extracted.
(2) The plasmid pUSapoEO (1 µg) was digested with ScaI (1 u) in Buffer H (10 µl) at 37°C for 2 hours. The digestion mixture was treated with alkaline phosphatase (1 u) at 60°C for one hour to remove 5′-terminal phosphate groups. The mixture was then treated with water-saturated phenol to remove proteins followed by treatment with ether to remove the phenol. The resulting mixture was dialyzed against water, evaporated to dryness and dissolved in water (10 µl) to yield an aqueous solution of Fragment II.
(3) A variant primer (50 pmol) of 41 bases as shown in Fig. 8 was phosphorylated at 5′-terminal by the treatment with T4 polynucleotide kinase (1 u) in Buffer K (10 µl) at 37°C for one hour.
(4) Fragment I (0.05 pmol), Fragment II (0.05 pmol) and 5′-phosphorylated primer (45 pmol) were mixed with 12 µl of 5X conc. polymerase-ligase buffer (0.5 M sodium chloride, 32.5 mM Tris-HCl, pH 7.5, 40 mM magnesium chloride and 5 mM beta-mercaptoethanol). The total volume was adjusted to 34.8 µl. The mixture was boiled at 100°C for 3 minutes, immediately after which it was placed into a thermostat at 30°C and allowed to stand for 30 minutes. The mixture was allowed to stand at 4°C for 30 minutes and then on ice for 10 minutes. Thus, a heteroduplex was formed.
(5) An aqueous solution (11.6 µl) containing the heteroduplex was mixed with 2.5 mM 4-deoxynucleotide triphosphate (2 µl), 10 mM ATP (2 µl), Klenow enzyme (2 u) and T4 DNA ligase (0.5 u). The total volume was adjusted to 20 µl. The mixture was reacted at 12.5°C for 16 hours to cyclize the DNA.

An aqueous solution (2 µl) containing the cyclized DNA was used to transform E. coli JM109 in conventional manners. Plasmids were isolated and purified from the transformants by conventional procedures. The plasmid DNAs were checked by digesting them with BglII and electrophoresing on 0.8% agarose gel. Thus, the cleaved plasmid was isolated as a desired variant plasmid.

The isolated variant plasmid was often contaminated with the original wild-type plasmid. The variant plasmid was again used to transform E. coli JM109 to purify the variant plasmid. Thus, purified plasmid pUSapoEBH was obtained.

### IV) Construction of Plasmid pNapoE and Secretory Production of Apolipoprotein E in E. coli

The plasmid pOFAapoE (10 µg) was digested with HindIII (10 u) in Buffer M (100 µl) at 37°C for 2 hours. The reaction mixture was subjected to 5% acrylamide gel electrophoresis and stained with 0.05% ethidium bromide. About 110 bp HindIII tac promoter fragment was excised and extracted from the gel.

On the other hand, pUSapoEBH (1 µg) was digested with HindIII (1 u) in Buffer M (10 µl) at 37°C for 2 hours. The reaction mixture was mixed with E. coli alkaline phosphatase (1 u) and incubated at 65°C for one hour. Water-saturated phenol was used to inactivate the enzyme and the phenol was removed by ether. The resulting mixture was dialyzed against water and evaporated to dryness.

The resulting plasmid DNA was mixed with the HindIII tac promoter fragment (0.1 pmol) and treated with T4 DNA ligase (1 u) in Buffer L (10 µl) at 4°C for 16 hours to ligate to each other.

The ligation mixture (5 µl) was used to transform E. coli JM109. Plasmids were isolated and purified from transformants. The resulting plasmid DNAs were checked by digestion with HindIII and other restriction enzymes. Thus, a desired plasmid pNapoE was obtained (Fig. 7D).

E. coli strains retaining the plasmid pNapoE was cultivated under the conditions similar to those used in Example 1. It was showed that natural-type human apolipoprotein E was produced and secreted.

### EXAMPLE 3:

The plasmid pOFAapoE constructed in Example 1 was digested with BamHI and BglII and the fused gene of OmpF signal peptide and apoE gene was isolated and purified by 5% polyacrylamide gel electrophoresis in conventional manners. This fused gene fragment was inserted into the BglII-cleaved site of pOFAapoE. Thus, a plasmid pOFAapoE₂ was obtained into which two fused gene fragments of OmpF signal peptide and apoE gene were introduced (Fig. 9).

Similarly, the plasmid pNapoE constructed in Example 2 was digested with BamHI and BglII and the fused gene of natual apoE signal peptide and apoE gene was isolated and purified by 5% polyacrylamide gel electrophoresis in conventional manners. This fused gene fragment was inserted into the BglII-cleaved site of pNapoE. Thus, a plasmid pNapoE₂ was obtained into which two fused gene fragments of apoE signal peptide and apoE gene were introduced (Fig. 10).

### EXAMPLE 4: Pharmacological Activities of Apolipoprotein E Produced by E. coli

The plasmid pOFAapoE₂ constructed in Example 3 was used to transform E. coli cells and the transformants were cultured to produce apolipoprotein E. The produced apolipoprotein E was isolated and purified in conventional manners. On the other hand, natural apolipoprotein E was obtained from blood samples according to the conventional methods: R.E. Stephans, Anal. Biochem., 65, 369 (1975).

Using these apolipoprotein E products, their abilities to bind to LDL receptors and to form complexes with phospholipids were measured according to the method of T.L. Innerarity et al.: J. Biol. Chem., 254, 4186 (1979). Both proteins showed equivalent activities in these tests.

As seen from the foregoing description, natural-type human apolipoprotein E-like proteins can be efficiently produced according to the present invention. When these proteins, including apolipoproteins E2, E3 and E4, are used as antigens to prepare anti-sera by conventional procedures, such anti-sera can be employed to detect the deficiency in normal-type apolipoprotein E3 or the presence of abnormal-type apolipoprotein E2 or E4. Further, apolipoprotein E3 produced according to the present invention can be utilized as anti-hyperlipidemia and anti-arteriosclerosis agenst.

## Claims

1. A process for the production of a natural human apolipoprotein E or a natural human apolipoprotein E-like protein having physiological activities similar to those of the natural human apolipoprotein E which comprises transforming a host microorganism, preferentially E. coli, with an expression vector into which a first DNA fragment coding for a secretory signal peptide and a second DNA fragment coding for the human apolipoprotein E or human apolipoprotein E-like protein are introduced downstream from a promoter; culturing the transformant; and collecting the natural human apolipoprotein E or natural human apolipoprotein E-like protein secreted in the supernatant.

2. The process of claim 1, wherein the DNA fragment coding for a secretory signal peptide is derived from Escherichia coli.

3. The process of claim 1, wherein the secretory signal peptide is the signal peptide for OmpF, OmpC, OmpA, a lipoprotein, alkaline phosphatase, AmpC-lactamase, or TEM beta-lactamase.

4. The process of claim 3, wherein the secretory signal peptide is OmpF or OmpC signal peptide.

5. The process of claim 1, wherein a DNA sequence in the vicinity of the initiation codon for the encoded signal peptide in the DNA fragment coding for a secretory signal peptide is rich in adenine and thymine.

6. The process of claim 5, wherein the DNA sequence in the vicinity of the initiation codon in the DNA fragment coding for a signal peptide is represented by 5′-AAGCTTATGATGAAG-3′, 5′-AAATTTATGAAG-3′, 5′-AAGCTTATGATGAAA-3′, 5′-AAATTTATGAAA-3′, 5′-AAGCTTATGGTT-3′, 5′-AAGCTTATGGTA-3′, 5′-AAATTTATGGTT-3′, or 5′-AAATTTATGGTA-3′.

7. The process of claim 1, wherein two or more promoters are linked in tandem.

8. The process of claim 1, wherein the promoter is tac or pac promoter.

9. The process of claim 1, wherein the expression vector has a ribosome binding region derived from lpp, trp, lac or lambda-CII gene.

10. The process of claim 9, wherein the expression vector has the lac-derived ribosome binding region.

11. The process of claim 1, wherein the expression vector has tac promoter and the lac-derived ribosome binding region.

12. The process of claim 1, wherein the expression vector has the ribosome binding region represented by 5′-AGGAGGTTT-3′, 5′-AGGAGGGTTT-3′, 5′-AGGAAACTGA-3′, 5′-TGGATTATTC-3′, or 5′-AGGAGGTATA-3′.

13. An expression vector in which two or more genes coding for a fused protein of a secretory signal peptide and human apolipoprotein E or a human apolipoprotein E-like protein having physiological activities similar to those of the human apolipoprotein E are linked in tandem, and said genes coding for said fused protein are under control of a promoter.

14. The expression vector of claim 13, wherein the promoter is tac or pac promoter.

15. The expression vector of claim 13, wherein a DNA fragment coding for the secretory signal peptide is derived from Escherichia coli.

16. The expression vector of claim 13, wherein the secretory signal peptide is the signal peptide for OmpF, OmpC, OmpA, a lipoprotein, alkaline phosphatase, AmpC-lactamase, or TEM beta-lactamase.

17. The expression vector of claim 16, wherein the secretory signal peptide is OmpF or OmpC signal peptide.

18. The expression vector of claim 13, wherein a DNA sequence in the vicinity of the initiation codon for the encoded signal peptide in the DNA fragment coding for the secretory signal peptide is rich in adenine and thymine.

19. The expression vector of claim 18, wherein the DNA sequence in the vicinity of the initiation codon in the DNA fragment coding for the signal peptide is represented by 5′-AAGCTTATGATGAAG-3′, 5′-AAATTTATGAAG-3′, 5′-AAGCTTATGATGAAA-3′, 5′-AAATTTATGAAA-3′, 5′-AAGCTTATGGTT-3′, 5′-AAGCTTATGGTA-3′, 5′-AAATTTATGGTT-3′, or 5′-AAATTTATGGTA-3′.

20. The expression vector of claim 13, wherein two or more promoters are linked in tandem.

21. The expression vector of claim 20, wherein the promoter is tac or pac promoter.

22. The expression vector of claim 13, which has a ribosome binding region derived from lpp, trp, lac or lambda-CII gene.

23. The expression vector of claim 22, which has the lac-derived ribosome binding region.

24. The expression vector of caim 13, which has tac promoter and the lac-derived ribosome binding region.

25. The expression vector of claim 22, wherein the ribosome binding region is represented by 5′-AGGAGGTTT-3′, 5′-AGGAGGGTTT-3′, 5′-AGGAAACTGA-3′, 5′-TGGATTATTC-3′, or 5′-AGGAGGTATA-3′.

26. The expression vector of claim 13, which is pOFAapoE₂.

27. The expression vector of claim 13, which is pNapoE₂.

28. An expression vector pOFAapoE.

29. An expression vector pNapoE.

30. Escherichia coli transformed with an expression vector of any one of claims 13 to 29.

## Patentansprüche

1. Verfahren zur Herstellung eines natürlichen menschlichen Apolipoproteins E oder eines zu natürlichem menschlichem Apolipoprotein E ähnlichen Proteins mit einer physiologischen Aktivität, die der von natürlichem menschlichem Apolipoprotein E entspricht, wobei das Verfahren das Transformieren eines Wirtsmikroorganismus, vorzugsweise E. coli, mit einem Expressionsvektor, in den ein erstes DNA-Fragment, das für ein Absonderungssignalpeptid codiert, und ein zweites DNA-Fragment, das für menschliches Apolipoprotein E oder für ein zu menschlichem Apolipoprotein E ähnliches Protein codiert, stromabwärts von einem Promotor eingeführt sind; das Züchten des Transformanten; und die Gewinnung des natürlichen menschlichen Apolipoproteins E oder des zu natürlichem menschlichem Apolipoprotein E ähnlichen Proteins, das in den Überstand abgesondert ist, umfaßt.

2. Verfahren nach Anspruch 1, wobei das DNA-Fragment, das für das Absonderungssignalpeptid codiert, aus Escherichia coli abgeleitet ist.

3. Verfahren nach Anspruch 1, wobei es sich bei dem Absonderungssignalpeptid um das Signalpeptid für OmpF, OmpC, OmpA, ein Lipoprotein, alkalische Phosphatase, AmpC-Lactamase oder TEM-β-Lactamase handelt.

4. Verfahren nach Anspruch 3, wobei es sich bei dem Absonderungssignalpeptid um das OmpF- oder OmpC-Signalpeptid handelt.

5. Verfahren nach Anspruch 1, wobei eine DNA-Sequenz in der Nähe des Initiationscodons für das codierte Signalpeptid im DNA-Fragment, das für das Absonderungssignalpeptid codiert, reich an Adenin und Thymin ist.

6. Verfahren nach Anspruch 5, wobei die DNA-Sequenz in der Nähe des Initiationscodons im DNA-Fragment, das für das Signalpeptid codiert, durch 5'-AAGCTTATGATGAAG-3', 5'-AAATTTATGAAG-3', 5'-AAGCTTATGATGAAA-3', 5'-AAATTTATGAAA-3', 5'-AAGCTTATGGTT-3', 5'-AAGCTTATGGTA-3', 5'-AAATTTATGGTT-3' oder 5'-AAATTTATGGTA-3' dargestellt wird.

7. Verfahren nach Anspruch 1, worin zwei oder mehr Promotoren in einer Tandem-Anordnung verknüpft sind.

8. Verfahren nach Anspruch 1, wobei es sich bei dem Promotor um den tac- oder den pac-Promotor handelt.

9. Verfahren nach Anspruch 1, wobei der Expressionsvektor eine Ribosomen-Bindungsregion aufweist, die aus dem lpp-, dem trp-, dem lac- oder dem lambda-CII-Gen abgeleitet ist.

10. Verfahren nach Anspruch 9, wobei der Expressionsvektor die aus lac abgeleitete Ribosomen-Bindungsregion aufweist.

11. Verfahren nach Anspruch 1, wobei der Expressionsvektor den tac-Promotor und die aus lac abgeleitete Ribosomen-Bindungsregion aufweist.

12. Verfahren nach Anspruch 1, wobei der Expressionsvektor die Ribosomen-Bindungsregion aufweist, die durch 5'-AGGAGGTTT-3', 5'-AGGAGGGTTT-3', 5'-AGGAAACTGA-3', 5'-TGGATTATTC-3' oder 5'AGGAGGTATA-3' dargestellt wird.

13. Expressionsvektor, in dem zwei oder mehr Gene, die für ein Fusionsprotein aus einem Absonderungssignalpeptid und menschlichem Apolipoprotein E oder einem zu menschlichem Apolipoprotein E ähnlichen Protein, das eine physiologische Aktivität aufweist, die menschlichem Apolipoprotein E entspricht, in einer Tandem-Anordnung verknüpft sind, und sich die Gene, die für das Fusionsprotein codieren, unter der Steuerung eines Promotors befinden.

14. Expressionsvektor nach Anspruch 13, wobei es sich bei dem Promotor um den tac- oder den pac-Promotor handelt.

15. Expressionsvektor nach Anspruch 13, wobei ein DNA-Fragment, das für das Absonderungssignalpeptid codiert, aus Escherichia coli abgeleitet ist.

16. Expressionsvektor nach Anspruch 13, wobei es sich bei dem Absonderungssignalpeptid um das Signalpeptid für OmpF, Ompc, OmpA, ein Lipoprotein, alkalische Phosphatase, AmpC-Lactamase oder TEM-β-Lactamase handelt.

17. Expressionsvektor nach Anspruch 16, wobei es sich bei dem Absonderungssignalpeptid um das OmpF- oder OmpC-Signalpeptid handelt.

18. Expressionsvektor nach Anspruch 13, wobei eine DNA-Sequenz in der Nähe des Initiationscodons für das codierte Signalpeptid im DNA-Fragment, das für das Absonderungssignalpeptid codiert, reich an Adenin und Thymin ist.

19. Expressionsvektor nach Anspruch 18, wobei die DNA-Sequenz in der Nähe des Initiationscodons im DNA-Fragment, das für das Signalpeptid codiert, durch 5'-AAGCTTATGATGAAG-3', 5'-AAATTTATGAAG-3', 5'-AAGCTTATGATGAAA-3', 5'-AAATTTATGAAA-3', 5'-AAGCTTATGGTT-3', 5'-AAGCTTATGGTA-3', 5'-AAATTTATGGTT-3' oder 5'-AAATTTATGGTA-3' dargestellt wird.

20. Expressionsvektor nach Anspruch 13, wobei zwei oder mehr Promotoren in einer Tandem-Anordnung verknüpft sind.

21. Expressionsvektor nach Anspruch 20, wobei es sich bei dem Promotor um den tac- oder den pac-Promotor handelt.

22. Expressionsvektor nach Anspruch 13, der eine Ribosomen-Bindungsregion aufweist, die aus dem lpp-, dem trp-, dem lac- oder dem lambda-CII-Gen abgeleitet ist.

23. Expressionsvektor nach Anspruch 22, der die aus lac abgeleitete Ribosomen-Bindungsregion aufweist.

24. Expressionsvektor nach Anspruch 13, der den tac-Promotor und die aus lac abgeleitete Ribosomen-Bindungsregion aufweist.

25. Expressionsvektor nach Anspruch 22, wobei die Ribosomen-Bindungsregion durch 5'-AGGAGGTTT-3', 5'-AGGAGGGTTT-3', 5'-AGGAAACTGA-3', 5'-TGGATTATTC-3' oder 5'AGGAGGTATA-3' dargestellt wird.

26. Expressionsvektor nach Anspruch 13, wobei es sich um pOFAapoE₂ handelt.

27. Expressionsvektor nach Anspruch 13, wobei es sich um pNapoE₂ handelt.

28. Expressionsvektor pOFAapoE.

29. Expressionsvektor pNapoE.

30. Escherichia coli, transformiert mit einem Expressionsvektor nach einem der Ansprüche 13 bis 29.

## Revendications

1. Procédé pour la production d'une apolipoprotéine E humaine naturelle ou d'une protéine analogue à une apolipoprotéine E humaine naturelle ayant des activités physiologiques similaires à celles de l'apolipoprotéine E humaine naturelle, qui comprend la transformation d'un microorganisme hôte, de préférence E. coli, avec un vecteur d'expression dans lequel un premier fragment d'ADN codant pour un peptide signal sécrétoire et un second fragment d'ADN codant pour l'apolipoprotéine E humaine naturelle ou la protéine analogue à une apolipoprotéine E humaine naturelle, sont introduits en aval d'un promoteur; la culture du transformant; et la collection de l'apolipoprotéine E humaine naturelle ou de la protéine analogue à une apolipoprotéine E humaine naturelle sécrétée dans le surnageant.

2. Procédé suivant la revendication 1, dans lequel le fragment d'ADN codant pour un peptide signal sécrétoire provient de Escherichia coli.

3. Procédé suivant la revendication 1, dans lequel le peptide signal sécrétoire est le peptide signal pour OmpF, OmpC, OmpA, une lipoprotéine, une phosphatase alcaline, une AmpC-lactamase ou une TEM béta-lactamase.

4. Procédé suivant la revendication 3, dans lequel le peptide signal sécrétoire est le peptide signal pour OmpF ou OmpC.

5. Procédé suivant la revendication 1, dans lequel une séquence d'ADN au voisinage du codon d'initiation pour le peptide signal codé dans le fragment d'ADN codant pour un peptide signal sécrétoire, est riche en adénine et thymine.

6. Procédé suivant la revendication 5, dans lequel la séquence d'ADN au voisinage du codon d'initiation dans le fragment d'ADN codant pour un peptide signal, est représentée par 5'-AAGCTTATGATGAAG-3', 5'-AAATTTATGAAG-3', 5'-AAGCTTATGATGAAA-3', 5'-AAATTTATGAAA-3', 5'-AAGCTTATGGTT-3', 5'-AAGCTTATGGTA-3', 5'-AAATTTATGGTT-3' ou 5'-AAATTTATGGTA-3'.

7. Procédé suivant la revendication 1, dans lequel deux ou plusieurs promoteurs sont liés en tandem.

8. Procédé suivant la revendication 1, dans lequel le promoteur est le promoteur *tac* ou *pac*.

9. Procédé suivant la revendication 1, dans lequel le vecteur d'expression a une région de liaison de ribosome provenant de *lpp, trp, lac* ou du gène lambda-CII.

10. Procédé suivant la revendication 9, dans lequel le vecteur d'expression a la région de liaison de ribosome dérivé de *lac.*

11. Procédé suivant la revendication 1, dans lequel le vecteur d'expression a le promoteur *tac* et la région de liaison de ribosome dérivée de *lac*.

12. Procédé suivant la revendication 1, dans lequel le vecteur d'expression a la région de liaison de ribosome représentée par 5'-AGGAGGTTT-3'_{,} 5'-AGGAGGGTTT-3', 5'-AGGAAACTGA-3', 5'-TGGATTATTC-3' ou 5'-AGGAGGTATA-3'.

13. Vecteur d'expression dans lequel deux ou plusieurs gènes codant pour une protéine fusionnée d'un peptide signal sécrétoire et d'une apolipoprotéine E humaine naturelle ou d'une protéine analogue à une apolipoprotéine E humaine naturelle ayant des activités physiologiques similaires à celles de l'apolipoprotéine E humaine naturelle, sont liés en tandem, et ces gènes codant pour cette protéine fusionnée sont sous le contrôle d'un promoteur.

14. Vecteur d'expression suivant la revendication 13, dans lequel le promoteur est le promoteur *tac* ou *pac*.

15. Vecteur d'expression suivant la revendication 13, dans lequel un fragment d'ADN codant pour le peptide signal sécrétoire provient de Escherichia coli.

16. Vecteur d'expression suivant la revendication 13, dans lequel le peptide signal sécrétoire est le peptide signal pour OmpF, OmpC, OmpA, une lipoprotéine, une phosphatase alcaline, une AmpC-lactamase ou une TEM béta-lactamase.

17. Vecteur d'expression suivant la revendication 16, dans lequel le peptide signal sécrétoire est le peptide signal pour OmpF ou OmpC.

18. Vecteur d'expression suivant la revendication 13, dans lequel une séquence d'ADN au voisinage du codon d'initiation pour le peptide signal codé dans le fragment d'ADN codant pour un peptide signal sécrétoire, est riche en adénine et thymine.

19. Vecteur d'expression suivant la revendication 18, dans lequel la séquence d'ADN au voisinage du codon d'initiation dans le fragment d'ADN codant pour le peptide signal, est représentée par 5'-AAGCTTATGATGAAG-3', 5'-AAATTTATGAAG-3', 5'-AAGCTTATGATGAAA-3', 5'-AAATTTATGAAA-3', 5'-AAGCTTATGGTT-3', 5'-AAGCTTATGGTA-3', 5'-AAATTTATGGTT-3' ou 5'-AAATTTATGGTA-3'.

20. Vecteur d'expression suivant la revendication 13, dans lequel deux ou plusieurs promoteurs sont liés en tandem.

21. Vecteur d'expression suivant la revendication 13, dans lequel le promoteur est le promoteur *tac* ou *pac.*

22. Vecteur d'expression suivant la revendication 13, qui a une région de liaison de ribosome provenant de *lpp, trp, lac* ou du gène lambda-CII.

23. Vecteur d'expression suivant la revendication 22, qui a la région de liaison de ribosome dérivée de *lac.*

24. Vecteur d'expression suivant la revendication 13, qui a le promoteur *tac* et la région de liaison de ribosome dérivée de *lac.*

25. Vecteur d'expression suivant la revendication 22, dans lequel la région de liaison de ribosome est représentée par 5'-AGGAGGTTT-3', 5'-AGGAGGGTTT-3', 5'-AGGAAACTGA-3', 5'--TGGATTATTC-3' ou 5'-AGGAGGTATA-3'.

26. Vecteur d'expression suivant la revendication 13, qui est pOFAapoE₂.

27. Vecteur d'expression suivant la revendication 13, qui est pNapoE₂.

28. Vecteur d'expression pOFAapoE.

29. Vecteur d'expression pNapoE.

30. Escherichia coli transformé avec un vecteur d'expression suivant l'une quelconque des revendications 13 à 29.
